# EUROPÄISCHE PATENTSCHRIFT

(11) **EP 0 538 680 B1**
(45) Veröffentlichungstag und Bekanntmachung des Hinweises auf die Patenterteilung: **21.09.1994**
(21) Anmeldenummer: 92117173.2
(22) Anmeldetag: 08.10.1992
(51) Int. Cl.: C07D 233/90

(54) **1,1'-Bis-(3-aminopropyl)-2,2'-diimidazol**
1,1'-bis-(3-aminopropyl)-2,2'-diimidazole
1,1'-bis-(3-aminopropyl)-2,2'-diimidazole

(30) Priorität: 22.10.1991 DE 4134808
(43) Veröffentlichungstag der Anmeldung: 28.04.1993
(73) Patentinhaber: BASF Aktiengesellschaft, 67063 Ludwigshafen (DE)
(72) Erfinder: Merger, Franz, Dr., W-6710 Frankenthal (DE); Ebel, Klaus, Dr., W-6704 Mutterstadt (DE); Brudermueller, Martin, Dr., W-6800 Mannheim 1 (DE)

(56) Entgegenhaltungen:
- EP-A- 0 239 246
- DE-A- 2 149 825
- H.F. MARK ET AL. 'Encyclopedia of polymer science and engineering Volume 6' 1986 , JOHN WILEY & SONS , NEW YORK
- CHEMICAL ABSTRACTS, vol. 89, 1978, Columbus, Ohio, US; abstract no. 139603e, M.S. HADDAD ET AL. Seite 683 ;

## Beschreibung

Die vorliegende Erfindung betrifft das neue 1,1'-Bis-(3-aminopropyl)-2,2'-diimidazol und Verfahren zu dessen Herstellung aus 2,2'-Diimidazol.

Aus "Handbook of thermoset plastics" Sidney M. Goodman, Verlag Noyes Publ. Partridge N.J. sind Imidazole bekannt, die sich als Epoxyhärter eignen.

Aus EP-A-239246 sind 1-Aminoethylimidazole bekannt, die sich als Epoxyhärter eignen.

Methoden zur aminierenden Hydrierung von Dinitrilen sind aus Houben-Weyl Bd.11/1, S.545 bis 569 bekannt. Alpha, omega-Dinitrile, wie Octamethylen-1,8-dinitril, lassen sich an Katalysatoren wie beispielsweise Raney-Nickel bei 100°C in Gegenwart von Wasserstoff und Ammoniak hydrieren (Org. Synthesis Vol.27, 18 (1947)).

Aus Org. Reactions Vol.5, 79 bis 135 (1942) ist die Cyanethylierung von N-Heterocyclen bekannt. Ferner ist aus Arzneim. Forschung 25(1), 9 bis 14 (1975) und aus der DE-A-21 49 825 die Mono- und Biscyanethylierung von 2,2'-Diimidazol in einer 0,1 bis 0,2 molaren Lösung in Dimethylformamid in Gegenwart von Natronlauge als Katalysator bekannt. Von Nachteil bei den zuvor beschriebenen Verfahren ist die niedrige Konzentration, d.h. der hohe Verbrauch an Lösungsmittel und die damit verbundene schlechte Raum-Zeit-Ausbeute.

Der vorliegenden Erfindung lag die Aufgabe zugrunde, neue 1,1'-Bis-(3-aminopropyl)-2,2'-diimidazole zu finden, die verbesserte Eigenschaften haben, und den zuvor genannten Nachteilen beim Verfahren zur Herstellung von Dinitrilen abzuhelfen.

Demgemäß wurden das neue 1,1'-Bis-(3-aminopropyl)-2,2'-diimidazol der Formel I 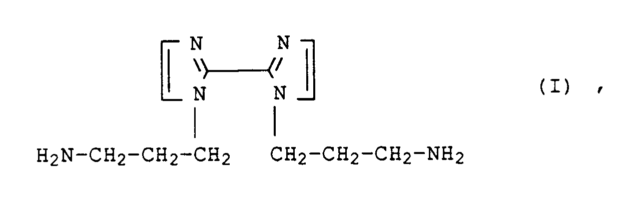
gefunden, sowie ein neues Verfahren zur Herstellung von 1,1'-Bis-(3-aminopropyl)-2,2'-diimidazol der Formel I, welches dadurch gekennzeichnet ist, daß man 2,2'-Diimidazol der Formel II, 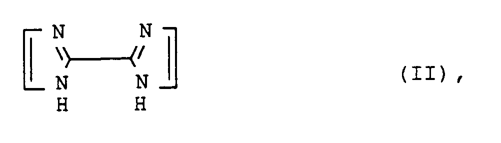
mit Acrylnitril der Formel III 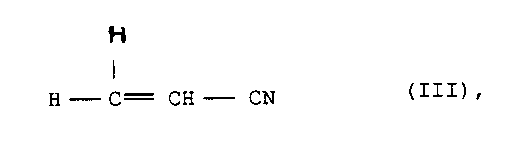
in Gegenwart von basischen Katalysatoren bei Temperaturen von 80 bis 150°C umsetzt und mit überschüssigem Wasserstoff in Gegenwart von Ammoniak an cobalt-, nickel-, ruthenium und/oder edelmetallhaltigen Katalysatoren bei Temperaturen von 40 bis 200°C und Drücken von 10 bis 200 bar hydriert.

Das erfindungsgemäße Verfahren läßt sich wie folgt durchführen:
a) 2,2'-Diimidazol II kann in Gegenwart eines basischen Katalysators gegebenenfalls in einem inerten Lösungsmittel vorgelegt und bei 80 bis 150°C, bevorzugt 80 bis 110°C umgesetzt werden. Der Druck bei dieser Reaktion ist unkritisch und liegt im allgemeinen bei 0,1 bis 50 bar, bevorzugt bei 0,5 bis 5 bar, besonders bevorzugt bei Atmosphärendruck (Normaldruck).
   Die Cyanethylierung von 2,2'-Diimidazol kann in Lösung oder in Suspensionsfahrweise durchgeführt werden, bevorzugt wird die Suspensionsfahrweise.
   Als inerte Lösungsmittel eignen sich beispielsweise Formamide wie Dimethylformamid, sowie aromatische Kohlenwasserstoffe wie Toluol oder die Xylol, bevorzugt Dimethylformamid. Üblicherweise werden 0,5 bis 5 l eines inerten Lösungsmittels pro Mol 2,2'-Diimidazol eingesetzt.
   Als Katalysatoren eignen sich basisch reagierende Verbindungen wie beispielsweise Alkali- oder Erdalkalihydroxide oder substituierte und unsubstituierte Ammoniumhydroxide, z.B. quartäre Ammoniumhydroxide wie Tetramethylammoniumhydroxid, Tetraethylammoniumhydroxid, Tetrabutylammoniumhydroxid und Tetra(2-hydroxyethyl)-ammoniumhydroxid in Mengen von 0,1 bis 20 Gew.%, bevorzugt 0,5 bis 10 Gew.% besonders bevorzugt 1 bis 5 Gew.% bezogen auf 2,2'-Diimidazol.
b) Die Hydrierung kann diskontinuierlich oder kontinuierlich z.B. in Rührautoklaven bei Reaktionstemperaturen von 40 bis 200°C, bevorzugt bei 70 bis 150°C und Drücken von 10 bis 200 bar Wasserstoffdruck, bevorzugt 50 bis 150 bar und in Gegenwart von 20 bis 200 Gew.-% flüssigem Ammoniak durchgeführt werden.
   Als Katalysatoren eignen sich cobalt-, nickel-, beispielsweise Raney-Cobalt oder Raney-Nickel, ruthenium- und/oder edelmetallhaltige Katalysatoren, beispielsweise Palladium auf Kohle.
   Geignete Lösungsmittel sind beispielsweise aliphatische Alkohole, z.B. C₁- bis C₂₀-Alkanole wie Methanol, Ethanol, Propanol oder Isopropanol sowie Ether wie beipielsweise Tetraahydrofuran oder Dioxan oder Gemische aus diesen. 2,2'-Diimidazol läßt sich aus Glyoxal und Ammoniak bzw. Ammoniumsalzen in Ausbeuten um 25 % herstellen (Liebigs Ann. Chem. 605 (1957), 32 bis 35). Die Herstellung aus Dibromacetaldehyd und Ammoniak verläuft mit Ausbeuten um 30%, die Synthese aus Hydroxyethylendiaminhydrochlorid mit einer Ausbeute von 60% (Khimiya Geterotsiklicheskikh Soedinenii 8 (1987), 1069 bis 1070). In 85% Ausbeute erhält man das 2,2'-Diimidazolbishydrogensulfat aus Glyoxalsulfat (Khimiya Geterot. Soedinenii 8(1987), 1069 bis 1070). Ausbeuten von über 80% erhält man durch Umsetzung von Diiminoglyoxalsäuremethylester mit Aminoacetaldehyddimethylacetal (Synthesis (1986), 336 bis 337).
   1,1'-Bis-(3-aminopropyl)-2,2'-diimidazol ist vielseitig verwendbar, u.a. als Epoxyhärter, als Diamin für neue Polyamide mit Imidazolstruktureinheiten, sowie als Komplexbildner auf zahlreichen Anwendungsgebieten, wie beispielsweise beim Korrosionsschutz.

### Beispiele

### Beispiel 1

In einem Rührreaktor wird eine Suspension aus 500 g 2,2'-Diimidazol (2,2 mol) und 20 g Tetramethylammoniumhydroxid in 1000 ml Dimethylformamid auf 100°C erwärmt und innerhalb von 10 Minuten 240 g Acrylnitril zudosiert. Man rührt 3 Stunden bei 75°C und kühlt die homogene Lösung mit Eis. Der ausgefallene Feststoff, 450 g 1,1'-Bis-(2-cyanethyl)-2,2'-diimidazol, wird abgesaugt und mit Ether gewaschen (Smp.: 210 bis 212°C, Ausbeute 84 %).

### Beispiel 2

Ein 2,5 l-Rührautoklav wird mit 400 g 1,1'-Bis-(2-cyanethyl)-2,2'-diimidazol, 80 g Raney-Cobalt, 250 ml flüssigem Ammoniak und 500 ml Tetrahydrofuran beschickt. Nach 10 Stunden bei 150°C und 150 bar Wasserstoff ist die Reaktion beendet. Nach Filtration und Destillation (Sdp.: 215°C/1 mbar) erhält man 330 g 1,1'-Bis-(3-aminopropyl)- 2,2'-diimidazol als farbloses Öl (Ausbeute 80 %).

## Patentansprüche

1. 1,1'-Bis-(3-aminopropyl)-2,2'-diimidazol der Formel I

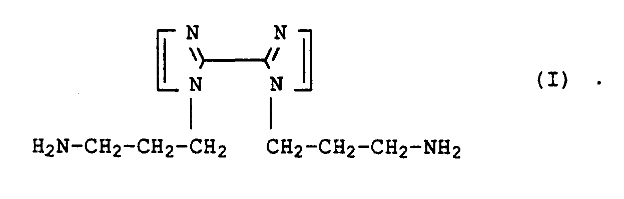

2. Verfahren zur Herstellung von 1,1'-Bis-(3-aminopropyl)-2,2'-diimidazol der Formel I 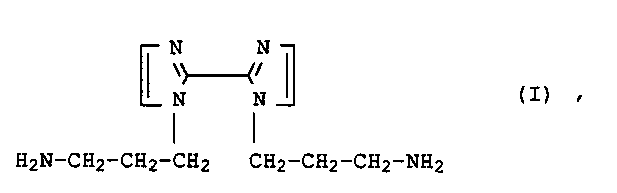 dadurch gekennzeichnet, daß man 2,2'-Diimidazol der Formel II, 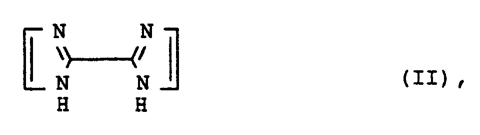 mit Acrylnitril der Formel III 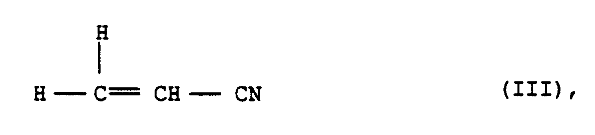 in Gegenwart von basischen Katalysatoren bei Temperaturen von 80 bis 150°C umsetzt und mit überschüssigem Wasserstoff in Gegenwart von Ammoniak an cobalt-, nickel-, ruthenium und/oder edelmetallhaltigen Katalysatoren bei Temperaturen von 40 bis 200°C und Drücken von 10 bis 200 bar hydriert.

3. Verfahren zur Herstellung von 1,1'-Bis-(3-aminopropyl)-2,2'-diimidazol der Formel I nach Anspruch 2, dadurch gekennzeichnet, daß man die Umsetzung mit Acrylnitril in der Suspensionfahrweise durchführt.

4. Verfahren zur Herstellung von 1,1'-Bis-(3-aminopropyl)-2,2'-diimidazol der Formel I nach Anspruch 1, dadurch gekennzeichnet, daß man die Hydrierung in C₁- bis C₂₀-Alkanolen und\oder acyclischen oder cyclischen Ethern durchführt.

## Claims

1. 1,1'-Bis(3-aminopropyl)-2,2'-diimidazole of the formula I

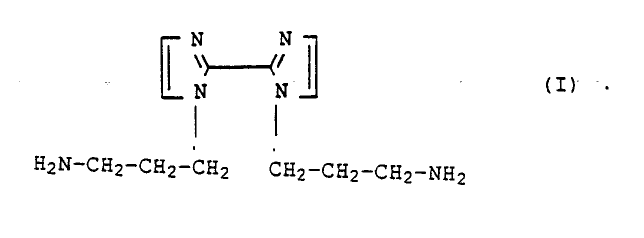

2. A process for preparing 1,1'-bis(3-aminopropyl)-2,2'-diimidazole of the formula I 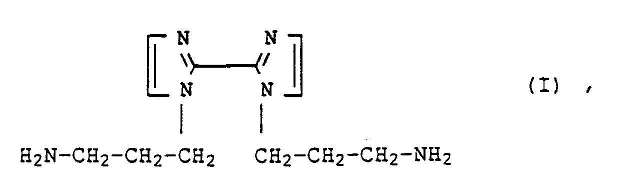 which comprises reacting 2,2'-diimidazole of the formula II 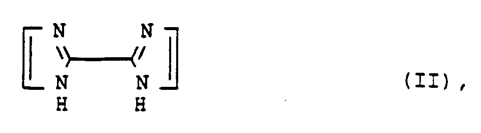 with acrylonitrile of the formula III 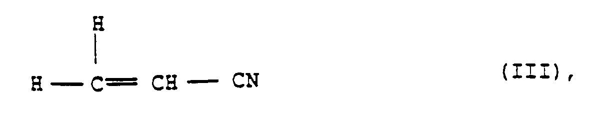 in the presence of basic catalysts at from 80 to 150°C, and hydrogenating with excess hydrogen in the presence of ammonia on catalysts which contain cobalt, nickel, ruthenium and/or noble metals at from 40 to 200°C under from 10 to 200 bar.

3. A process for preparing 1,1'-bis(3-aminopropyl)-2,2'-diimidazole of the formula I as claimed in claim 2, wherein the reaction with acrylonitrile is carried out as suspension reaction.

4. A process for preparing 1,1'-bis(3-aminopropyl)-2,2'-diimidazole of the formula I as claimed in claim 1, wherein the hydrogenation is carried out in C₁-C₂₀-alkanols and/or acyclic or cyclic ethers.

## Revendications

1. 1,1'-Bis-(3-aminopropyl)-2,2'-diimidazole de la formule I

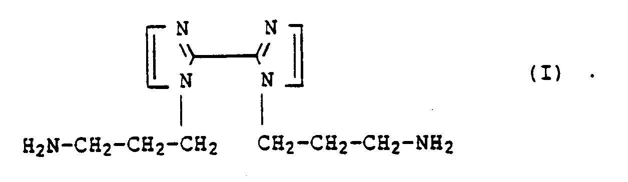

2. Procédé de préparation du 1,1'-bis-(3-aminopropyl)-2,2'-diimidazole de la formule I 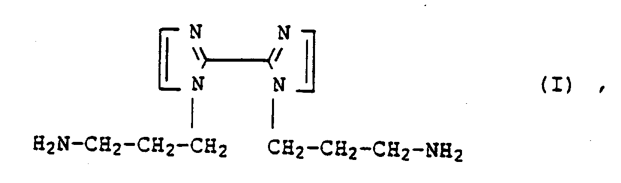 caractérisé en ce que l'on fait réagir le 2,2'-diimidazole de la formule II 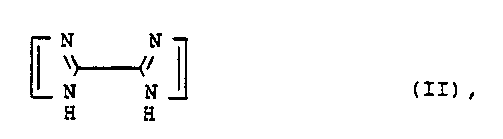 avec l'acrylonitrile de la formule III 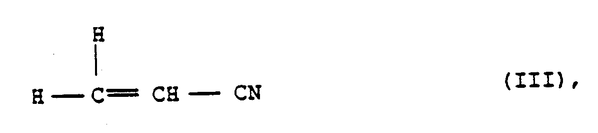 en présence de catalyseurs basiques et à des températures de 80 à 150°C et on procède à l'hydrogénation avec de l'hydrogène excédentaire, en présence d'ammoniac sur des catalyseurs contenant du cobalt, du nickel, du ruthénium et/ou des métaux nobles, à des températures de 40 à 200°C et sous des pressions de 1 à 200 bars.

3. Procédé de préparation du 1,1'-bis-(3-aminopropyl)-2,2'-diimidazole de la formule I suivant la revendication 2, caractérisé en ce que l'on entreprend la réaction avec l'acrylonitrile selon un mode en suspension.

4. Procédé de préparation du 1,1'-bis-(3-aminopropyl)-2,2'-diimidazole de la formule I suivant la revendication 1, caractérisé en ce que l'on entreprend l'hydrogénation dans des alcanols en C₁ à C₂₀ et/ou des éthers acycliques ou cycliques.
